# EUROPÄISCHE PATENTANMELDUNG

(11) **EP 0 649 882 A2**
(43) Veröffentlichungstag der Anmeldung: **26.04.1995**
(21) Anmeldenummer: 94116176.2
(22) Anmeldetag: 13.10.1994
(51) Int. Cl.: C09B 62/513, C09B 67/22, C09D 11/00

(54) **Wasserlösliche Trisazoverbindungen, Verfahren zu ihrer Herstellung und ihre Verwendung als Farbstoffe**

(30) Priorität: 18.10.1993 DE 4335408
(71) Anmelder: HOECHST AKTIENGESELLSCHAFT, D-65929 Frankfurt am Main (DE)
(72) Erfinder: v.d. Eltz, Andreas, Dr., D-60433 Frankfurt am Main (DE); Russ, Werner Hubert, Dr., D-65439 Flörsheim (DE); Bauer, Wolfgang, Dr., D-63477 Maintal (DE)

(57) **Zusammenfassung**

Es werden wasserlösliche Trisazoverbindungen der nachstehend angegebenen und definierten allgemeinen Formel (1) beschrieben, die sich als faserreaktive Farbstoffe zum Färben von Fasermaterialien auf Cellulosebasis natürlichen und synthetischen Ursprungs, wie z.B. Baumwolle und Viskoseseide, eignen und darüber hinaus als Farbstoffe für Tinten, die in den Ink-Jet-Druck eingesetzt werden können, Verwendung finden. 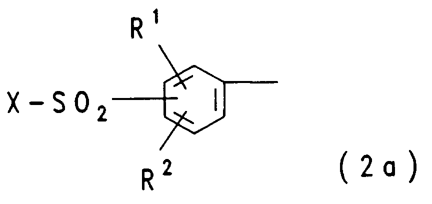
worin bedeuten: M ist Wasserstoff oder ein Alkalimetall, Y und Z, voneinander verschieden, sind jedes Hydroxy oder Amino, D¹ ist ein Rest der allgemeinen Formel (2a) und D² ein Rest der allgemeinen Formel (2b) 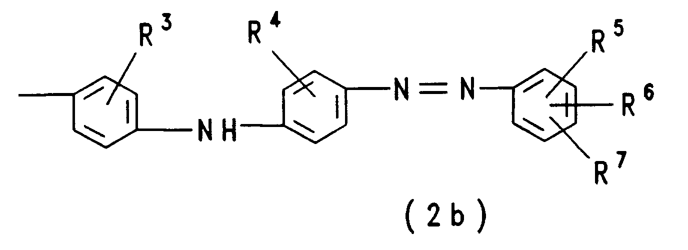
worin R¹ Wasserstoff oder niederes Alkoxy, R² Wasserstoff, niederes Alkyl oder niederes Alkoxy, X Vinyl oder β-Sulfatoethyl, R³, R⁴ und R⁷ jedes Wasserstoff oder Sulfo und R⁵ und R⁶ jedes Wasserstoff, Hydroxy oder Amino ist. Die Trisazoverbindungen liegen bevorzugt in Form von Mischungen vor.

## Beschreibung

Die Erfindung liegt auf dem technischen Gebiet der faserreaktiven Farbstoffe.

Aus der europäischen Patentanmeldungs-Veröffentlichung 0 471 233 sind bereits Trisazoverbindungen bekannt, die eine β-Hydroxyethylsulfonyl-Gruppe enthalten und die zum Färben von natürlichen und synthetischen Fasermaterialien, insbesondere zum Färben von Leder, und besonders zur Herstellung von Schreibtinten und Aufzeichnungsflüssigkeiten für das Tintenstrahl-Druckverfahren (Ink-Jet) eingesetzt werden können.

Mit der vorliegenden Erfindung wurden nunmehr neue Trisazoverbindungen gefunden, die eine faserreaktive Gruppe besitzen und die sich, insbesondere nach den Anwendungstechniken für faserreaktive Farbstoffe, ausgezeichnet zum Färben von Fasermaterialien auf Cellulosebasis natürlichen und synthetischen Ursprungs eignen, wie insbesondere zum Färben von Baumwolle, Viskoseseide und anderer hydroxygruppenhaltiger Celluloseregeneratfasern. Sie dienen außerdem als Farbstoffe für Tinten, insbesondere Tintenflüssigkeiten, die in den Ink-Jet-Druck eingesetzt werden können.

Die neuen Trisazoverbindungen entsprechen der allgemeinen Formel (1) 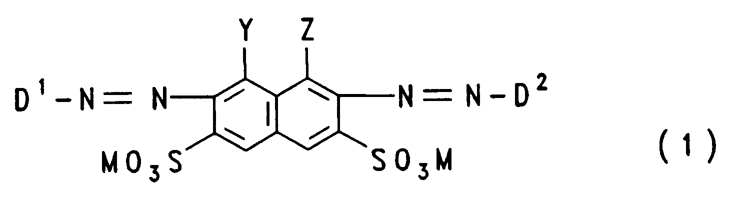
in welcher bedeuten:
- M: ist Wasserstoff oder ein Alkalimetall, wie Natrium, Kalium oder Lithium;
- Y: ist Hydroxy oder Amino und
- Z: ist Hydroxy oder Amino,
wobei im Falle von Y gleich Hydroxy Z gleich Amino und im Falle von Y gleich Amino Z gleich Hydroxy ist;
- D¹: ist ein Rest der allgemeinen Formel (2a) 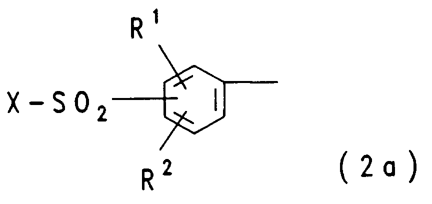 in welcher
- R¹: Wasserstoff oder Alkoxy von 1 bis 4 C-Atomen, wie Ethoxy und insbesondere Methoxy, ist,
- R²: Wasserstoff, Alkyl von 1 bis 4 C-Atomen, wie Ethyl und insbesondere Methyl, oder Alkoxy von 1 bis 4 C-Atomen, wie Ethoxy und insbesondere Methoxy, ist und
- X: Vinyl oder β-Sulfatoethyl ist;
- D²: ist ein Rest der allgemeinen Formel (2b) 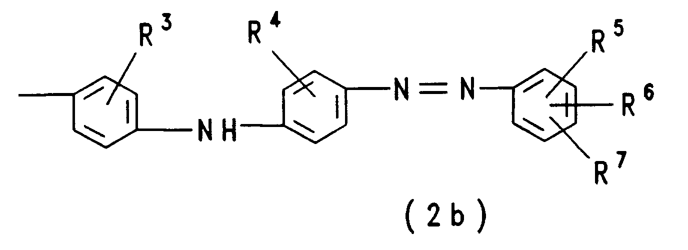 in welcher
- R³: Wasserstoff oder Sulfo ist,
- R⁴: Wasserstoff oder Sulfo ist,
- R⁵: Wasserstoff, Hydroxy oder Amino ist,
- R⁶: Wasserstoff, Hydroxy oder Amino ist und
- R⁷: Wasserstoff oder Sulfo ist.

In den obigen allgemeinen Formeln als auch in den nachfolgenden allgemeinen Formeln können die einzelnen Formelglieder im Rahmen ihrer Bedeutung zueinander gleiche oder voneinander verschiedene Bedeutungen besitzen. Die Gruppen "Sulfo" und "Sulfato" schließen sowohl deren Säureform als auch deren Salzform ein. Demgemäß bedeuten Sulfogruppen Gruppen entsprechend der allgemeinen Formel -SO₃M und Sulfatogruppen Gruppen entsprechend der allgemeinen Formel -OSO₃M , jeweils mit M der obengenannten Bedeutung.

Die Trisazoverbindungen der allgemeinen Formel (1) können sowohl als Einzelverbindungen als auch bevorzugt als Gemische vorliegen.

Die Gruppe X-SO₂- ist bevorzugt in meta- oder para-Stellung zur freien, zur Azogruppe hinführenden Bindung an den Benzolkern gebunden. Die Reste R⁵ und R⁶ stehen bevorzugt in meta-Stellung zueinander an den Benzolkern gebunden, wobei R⁵ bevorzugt Hydroxy und R⁶ bevorzugt Amino ist.

Insbesondere sind solche erfindungsgemäßen Trisazoverbindungen hervorzuheben, die den nachfolgend angegebenen und definierten allgemeinen Formeln (1A), (1B) und (1C) entsprechen, wobei diese bevorzugt als Mischung vorliegen und wobei wiederum bevorzugt das Mischungsverhältnis der Komponenten (1A), (1B) und (1C) in der Mischung zwischen 52 und 62 Gew.-% für die Komponente (1A), zwischen 34 und 44 Gew.-% für die Komponente (1B) und zwischen 3 und 7 Gew.-% für die Komponente (1C), bezogen auf 100 Gew.-Teile der Mischung aus diesen Komponenten, beträgt. 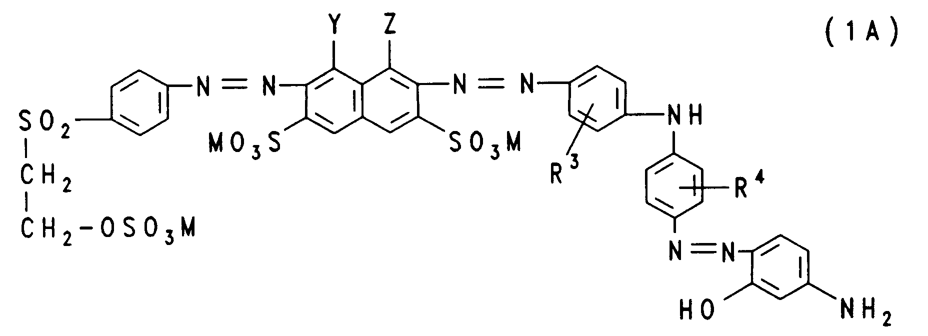 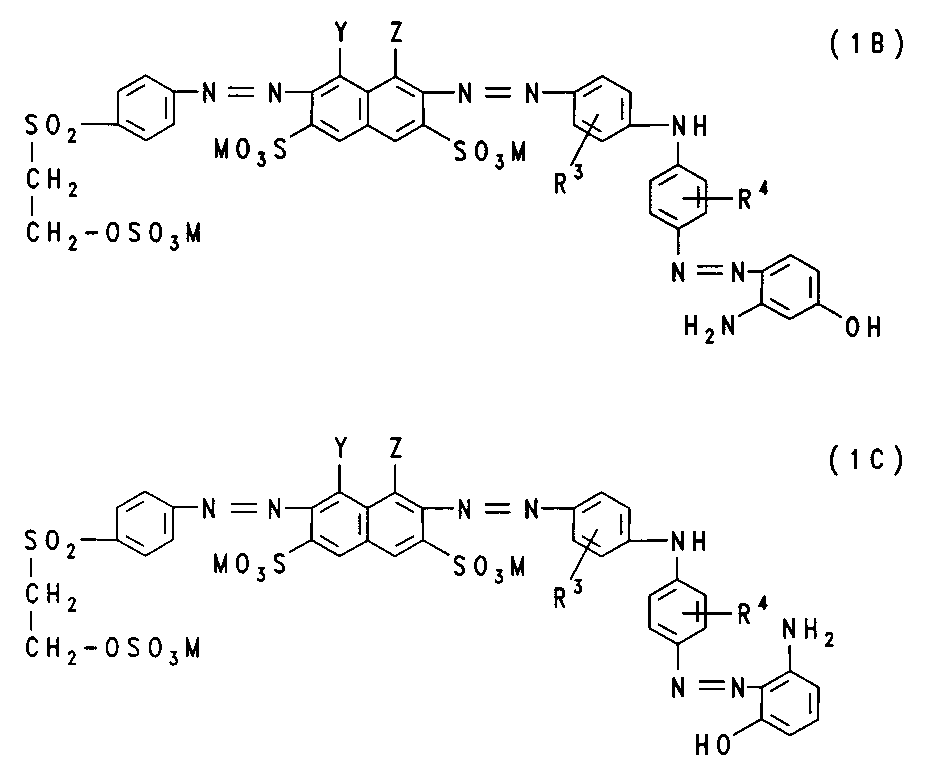
In diesen Formeln haben M, Y, Z, R³ und R⁴ die obengenannten Bedeutungen, wobei jedoch im Falle von R³ gleich Wasserstoff R⁴ gleich Sulfo ist und im Falle von R³ gleich Sulfo R⁴ gleich Wasserstoff ist.

Die erfindungsgemäßen Trisazoverbindungen und deren Mischungen eignen sich sehr gut als Farbstoffe für die anfangs genannten Einsatzgebiete. Sie zeigen beim Färben ein besonders günstiges Temperaturprofil und liefern insbesondere beim Färben oder Bedrucken der anfangs genannten Fasermaterialien bei Anwendung in einem Temperaturbereich zwischen 40 und 80°C Färbungen und Drucke mit gleicher Farbtiefe. Die erfindungsgemäßen Trisazoverbindungen und deren Mischungen liefern auf den genannten Fasermaterialien oder auch in Form der Tintenformulierungen auf den hierfür geeigneten Substraten Färbungen und Drucke von grünschwarzer bis neutral-grauer und neutral-schwarzer Farbnuance. Mit ihnen lassen sich ohne weitere Schwierigkeiten Färbungen und Drucke in gewünschten neutralen Grauabstufungen erhalten. Die erfindungsgemäß erhältlichen Färbungen und Drucke zeigen sehr gute allgemeine Echtheiten, wie zum Beispiel eine gute Lichtechtheit und gute Schweißlichtechtheiten; weiterhin weisen sie keinerlei Griffverhärtung im Druck auf. Die erfindungsgemäßen Trisazoverbindungen als auch die mit ihnen erhältlichen Färbungen und Drucke eignen sich weiterhin zum Einsatz im Ätzdruck. Die erfindungsgemäßen Trisazoverbindungen zeigen darüberhinaus eine außerordentliche Prozeßsicherheit.

Gegenstand der vorliegenden Erfindung ist deshalb auch die Verwendung der erfindungsgemäßen Trisazoverbindungen der allgemeinen Formel (1) und deren Mischungen zum Färben (einschließlich des Bedruckens) der anfangs genannten Fasermaterialien in an und für sich üblicher Verfahrensweise, insbesondere nach den Methoden der Applikation und Fixierung von faserreaktiven Farbstoffen auf Fasermaterialien, die zahlreich in der allgemeinen Fachliteratur und ebenso in der Patentliteratur beschrieben sind, indem man die Trisazoverbindung oder eine Mischung derselben im wäßrigen Medium auf das Fasermaterial appliziert und den oder die Farbstoffe mittels Wärme oder mittels einer alkalisch wirkenden Verbindung oder mittels beider Maßnahmen auf dem Fasermaterial fixiert.

Die Erfindung betrifft weiterhin Verfahren zur Herstellung der Trisazoverbindungen der allgemeinen Formel (1) und deren Mischungen. Sie sind dadurch gekennzeichnet, daß man ein auf üblichem Wege hergestelltes Diazoniumsalz eines Amins der allgemeinen Formel D¹-NH₂ mit D¹ der obengenannten Bedeutung und ein auf üblichem Wege hergestelltes Diazoniumsalz eines Amins der allgemeinen Formel D²-NH₂ mit D² der obengenannten Bedeutung mit der 1-Amino-8-hydroxy-naphthalin-3,6-disulfonsäure kuppelt, oder daß man ein Diazoniumsalz des Amins der allgemeinen Formel D¹-NH₂ mit D¹ der obengenannten Bedeutung und ein auf üblichem Wege hergestelltes Tetrazoniumsalz einer Aminoverbindung der allgemeinen Formel (3) 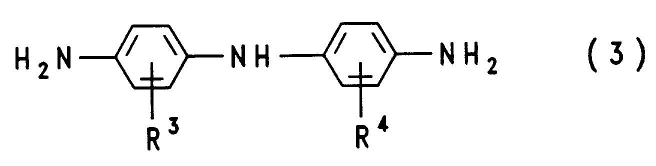
in welcher R³ und R⁴ die obengenannten Bedeutungen haben, mit 1-Amino-8-hydroxy-naphthalin-3,6-disulfonsäure kuppelt und das erhaltene Diazoniumsalz der Disazoverbindung der allgemeinen Formel (4) 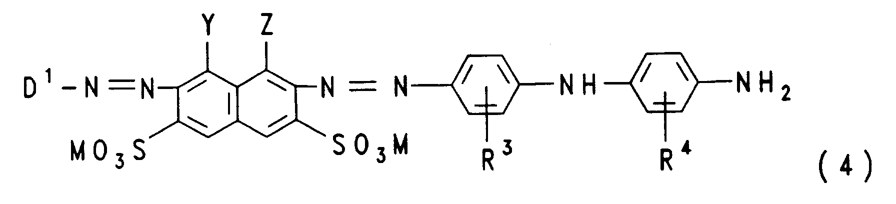
in welcher D¹, Y, Z, R³ und R⁴ die obengenannten Bedeutungen haben, mit einer Verbindung der allgemeinen Formel (5) 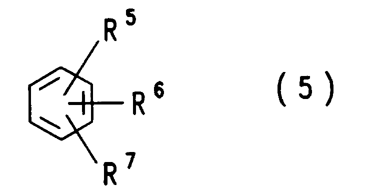
in welcher R⁵, R⁶ und R⁷ die obengenannten Bedeutungen haben, kuppelt.

Die Diazotierungsreaktionen verlaufen in üblicher und allgemein bekannter Verfahrensweise, vorzugsweise in wäßrigem, stark saurem, wie schwefelsaurem und vorzugsweise salzsaurem, Medium mittels Natriumnitrit bei einer Temperatur zwischen -5°C und + 15°C; bevorzugt liegt der pH-Wert des Diazotierungsmediums unterhalb 2. Die Kupplungsreaktionen zwischen den Diazoniumsalzen der allgemeinen Formeln D¹-NH₂ und D²-NH₂ bzw. eines Amins der allgemeinen Formel (3) 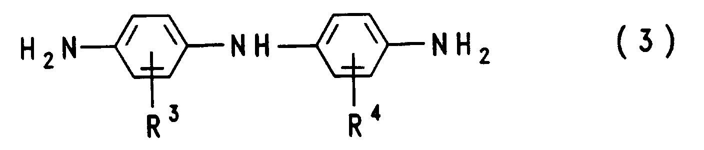
mit der 1-Amino-8-hydroxy-naphthalin-3,6-disulfonsäure verlaufen in altbekannter Weise: Im ersten Kupplungsschritt im stark sauren Medium, wie beispielsweise bei einem pH-Wert unterhalb von 2, erfolgt die Ankupplung in ortho-Stellung zur Aminogruppe und im zweiten Kupplungsschritt bei einem pH-Wert zwischen 3 und 7, bevorzugt zwischen 4 und 6,5, in ortho-Stellung zur Hydroxygruppe. Je nach dem, welches Amin in den ersten Kupplungsschritt eingesetzt wird, erhält man den oder die erfindungsgemäßen Trisazoverbindungen mit derjenigen Konstitution, bei der der Rest D¹ oder aber der Rest D² in ortho-Stellung zur Aminogruppe der Disulfo-amino-hydroxy-naphthalin-Kupplungskomponente gebunden ist.

Die Kupplungsreaktion zwischen dem Diazoniumsalz der Verbindung der allgemeinen Formel (4) 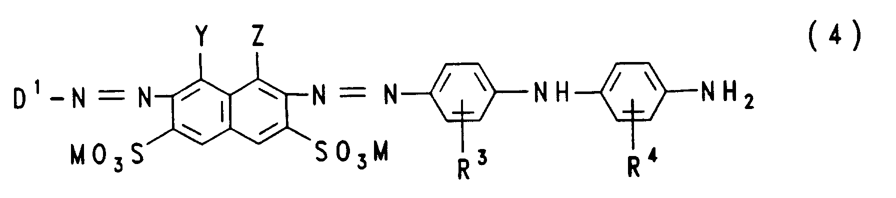
mit einem Aminophenol, einer Dihydroxy-benzol- oder Diamino-benzol-Verbindung erfolgt nach an und für sich bekannten Methoden innerhalb eines pH-Bereiches zwischen 4 und 7.

Sämtliche Kupplungsreaktionen werden bevorzugt in wäßrigem Medium durchgeführt, und zwar bei einer Temperatur zwischen 10 und 30°C.

Insbesondere bei der Herstellung von Mischungen der erfindungsgemäßen Trisazoverbindungen läßt sich die Herstellungsweise vereinfachen, wenn man die Synthese nicht in Einzelschritten, sondern in einem "Eintopf-Verfahren" durchführt, indem man die Diazoniumverbindungen des Amins der Formel D¹-NH₂ und des Amins der Formel D²-NH₂ bzw. der allgemeinen Formel (3) in stark saurem Reaktionsmedium mit der 1-Amino-8-hydroxy-naphthalin-3,6-disulfonsäure vermischt und zunächst die erste Kupplungsreaktion im stark sauren Medium durchführt, wobei in statistischen Anteilen sowohl die Diazokomponente des Restes D¹ als auch die Diazokomponente des Restes D² bzw. des entsprechenden Restes des Amins der allgemeinen Formel (3) in ortho-Stellung zur Aminogruppe des Aminonaphthols gebunden wird, und anschließend die zweite Kupplungsreaktion im angegebenen schwach sauren bis neutralen Bereich durchführt, wobei in statistischer Verteilung sowohl der Diazokomponentenrest D¹ und D² bzw. des Amins der allgemeinen Formel (3) in ortho-Stellung zur Hydroxygruppe des Aminonaphthols gebunden wird. Man kann diese Herstellungsweise auch in der Weise variieren, daß man die Amine der allgemeinen Formel D¹-NH₂ und D²-NH₂ bzw. das Amin der allgemeinen Formel (3) und die 1-Amino-8-hydroxy-naphthalin-3,6-disulfonsäure in dem neutralen bis stark sauren Reaktionsmedium miteinander vermischt und zunächst das Gemisch der diazotierbaren Aminoverbindungen, erforderlichenfalls nach Zugabe der nötigen Säure, gemeinsam diazotiert, wobei gleichzeitig der erste Kupplungsschritt ausgeführt wird, und daß man sodann in dem Reaktionsgemisch nach Einstellung eines schwach sauren bis neutralen pH-Wertes die zweite Kupplungsreaktion durchführt.

Die Abscheidung der erfindungsgemäßen Trisazoverbindung der allgemeinen Formel (1) bzw. deren Gemische aus den Syntheseansätzen erfolgt nach allgemein bekannten Methoden entweder durch Ausfällen aus dem Reaktionsmedium mittels Elektrolyten, wie beispielsweise Natriumchlorid oder Kaliumchlorid, oder durch Eindampfen der Reaktionslösung unter reduziertem Druck oder durch Sprühtrocknung, wobei der Syntheselösung eine Puffersubstanz, die einen pH-Wert zwischen 3 und 7 einzustellen und zu halten vermag, zugefügt werden kann.

Die nachfolgenden Beispiele dienen zur Erläuterung der Erfindung. Die in den Beispielen genannten Teile sind Gewichtsteile und die Prozentangaben stellen Gewichtsprozente dar, sofern nicht anders vermerkt. Gewichtsteile stehen zu Volumenteilen im Verhältnis von Kilogramm zu Liter.

Die für die erfindungsgemäßen Verbindungen angegebenen Absorptionsmaxima (λₘₐₓ) im sichtbaren Bereich wurden anhand ihrer Alkalimetallsalze in wäßriger Lösung ermittelt.

### Beispiel 1

171 Teile 4,4'-Diamino-diphenylamin-2-sulfonsäure werden in 300 Teile einer 31 %igen wäßrigen Salzsäure eingerührt; 156 Teile 2-Methoxy-5-(β-sulfatoethylsulfonyl)-anilin werden hinzugegeben. In dieser Mischung werden die beiden Aminoverbindungen durch Zugabe von 310 Teilen einer wäßrigen 5n-Natriumnitritlösung diazotiert. Danach wird überschüssiges Nitrit mittels Amidosulfonsäure zerstört und ein pH-Wert von 2 durch Zugabe von festem Natriumhydrogencarbonat eingestellt. Man gibt 139 Teile 1-Amino-8-hydroxy-naphthalin-3,6-disulfonsäure hinzu und führt die Kupplungsreaktion unter Einhaltung dieses pH-Wertes bei einer Temperatur von etwa 15°C während etwa 24 Stunden durch. Anschließend stellt man mittels Natriumcarbonat den pH-Wert auf 5 bis 6, rührt bei etwa 15°C noch drei Stunden nach, versetzt die erhaltene aminogruppenhaltige Disazoverbindung mit einer etwa 40°C warmen wäßrigen Lösung von 57,8 Teilen 3-Amino-phenol und führt die Kupplungsreaktion bei 15 bis 20°C und einem pH-Wert zwischen 5 und 6 während einer Zeit von zwei Tagen zu Ende.

Die erfindungsgemäßen Trisazoverbindungen werden in üblicher Weise isoliert, beispielsweise durch Ausfällen mittels Natriumchlorid und Filtration. Nach dem Trocknen wird ein elektrolytsalzhaltiges (natriumchloridhaltiges) Pulver erhalten, das die erfindungsgemäße Mischung von erfindungsgemäßen Trisazoverbindungen der allgemeinen Formeln (10A) bis (10C) und (11A) bis (11C) enthält: 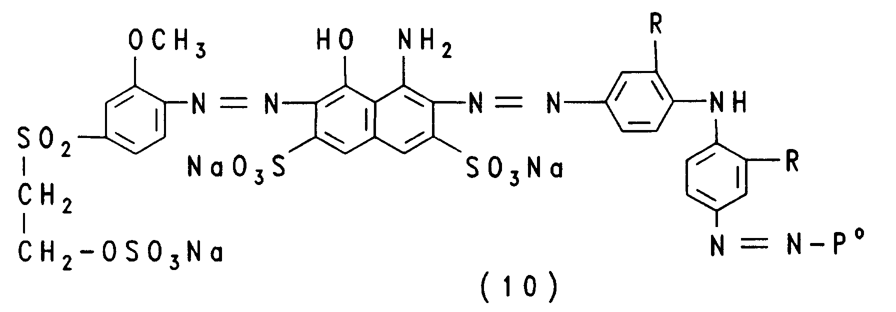 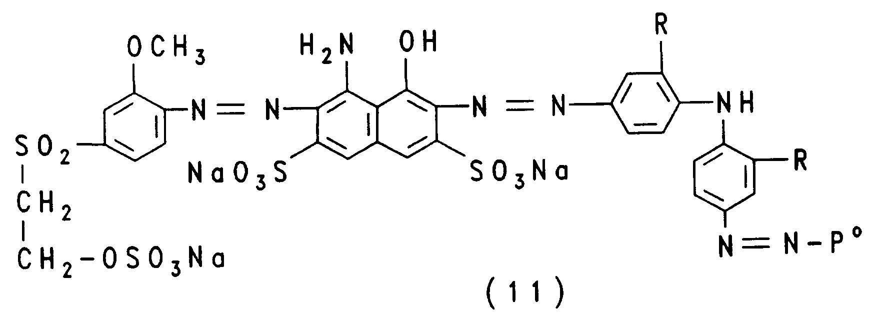
in welchen der eine Rest R Wasserstoff und der andere Rest R Sulfo ist und der Rest P^{o} die folgende Bedeutung besitzt:
für Formeln (10A) und (11A): 2-Hydroxy-4-amino-phenyl ;
für Formeln (10B) und (11B): 4-Hydroxy-2-amino-phenyl ;
für Formeln (10C) und (11C): 2-Hydroxy-6-amino-phenyl ,
und wobei ein Teil der Farbstoffe auch in der Vinylsulfonyl-Form vorliegt.

Diese Mischung, die in sichtbarem Bereich Absorptionsmaxima bei 485 nm und 628 nm aufweist, besitzt sehr gute faserreaktive Farbstoffeigenschaften und färbt beispielsweise nach den in der Technik üblichen bekannten Anwendungs- und Fixierverfahren die in der Beschreibung genannten Fasermaterialien, wie insbesondere Baumwolle, je nach Einsatz der Farbstoffmenge, in neutral-grauer bis neutral-schwarzer Nuance mit guten Echtheitseigenschaften, wie guten Licht- und Schweißlichtechtheiten.

### Beispiel 2

171 Teile 4,4'-Diamino-diphenylamin-2-sulfonsäure werden mit 181 Teilen 4-(β-Sulfatoethylsulfonyl)-anilin in 1500 Teilen Wasser gelöst und durch Zugabe von 310 Teilen einer wäßrigen 5n-Natriumnitritlösung und anschließend 300 Teilen einer 31 %igen wäßrigen Salzsäure bei einer Temperatur von etwa 5°C in üblicher Weise diazotiert. Überschüssige salpetrige Säure wird mittels Amidosulfonsäure zerstört und der pH-Wert danach mit festem 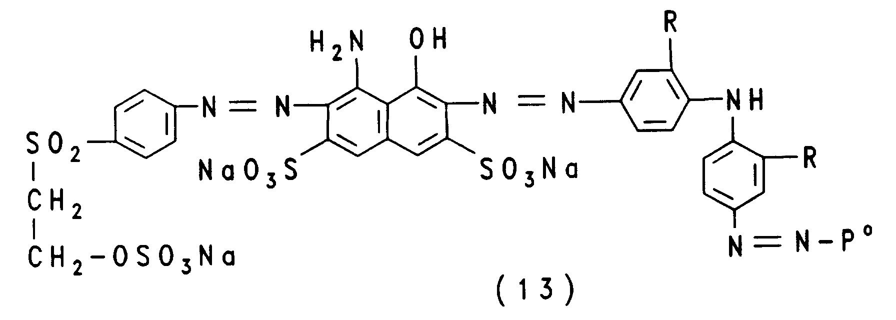
in welchen der eine Rest R Wasserstoff und der andere Rest R Sulfo ist und der Rest P^{o} die folgende Bedeutung besitzt:
für Formeln (12A) und (13A): 2-Hydroxy-4-amino-phenyl ;
für Formeln (12B) und (13B): 4-Hydroxy-2-amino-phenyl ;
für Formeln (12C) und (13C): 2-Hydroxy-6-amino-phenyl .

Diese Mischung der erfindungsgemäßen Trisazoverbindungen besitzt sehr gute faserreaktive Farbstoffeigenschaften. Mit ihr werden nach den in der Technik für faserreaktive Farbstoffe üblichen Anwendungs- und Fixierverfahren farbstarke Färbungen und Drucke in neutral-grauer Nuance erhalten.
Die Farbstoffmischung zeigt in wäßrigem Medium im sichtbaren Bereich ein Absorptionsmaximum bei 485 nm und bei 632 nm.

### Beispiel 3

139,4 Teile 4,4'-Diamino-diphenylamin-2-sulfonsäure werden in 500 Teilen Wasser angerührt; die Mischung wird anschließend mit 300 Teilen einer 31 %igen wäßrigen Salzsäure und 1000 Teilen Eis versetzt. Die Diazotierung des Diamino-diphenylamins erfolgt in üblicher Weise durch langsame Zugabe von 210 Teilen einer wäßrigen 5n-Natriumnitritlösung. Man rührt den Ansatz noch etwa 90 Minuten weiter, zerstört überschüssige salpetrige Säure mit Amidosulfonsäure, stellt mit Natriumacetat einen pH-Wert von 2 ein und gibt daraufhin langsam unter Einhaltung eines pH-Wertes von 7 eine Lösung von 139 Teilen 1-Amino-8-hydroxy-naphthalin-3,6-disulfonsäure in 250 Teilen Wasser hinzu. Man rührt den Ansatz bei einem pH-Wert von 2 bis 2,5 und einer Temperatur von 0 bis 5°C noch etwa 12 Stunden weiter und gibt sodann 139,2 Teile 4-(β-Sulfatoethylsulfonyl)-anilin in Form eines feingemahlenen Pulvers hinzu. Nachdem sich ein pH-Wert von 1,6 eingestellt hat, gibt man 100 Teile einer wäßrigen 5n-Natriumnitritlösung hinzu, rührt den Ansatz noch etwa eine Stunde bei 0 bis 5°C nach, zerstört sodann überschüssige salpetrige Säure mit Amidosulfonsäure, gibt Natriumcarbonat hinzu, bis ein pH-Wert von 5 bis 5,5 erhalten wird, und führt die zweite Kupplungsreaktion unter Einhaltung dieses pH-Wertes zu Ende.

Die erhaltene Disazoverbindung mit einer Diazoniumgruppe am Diphenylaminorest wird nunmehr mit einer warmen wäßrigen Lösung von 57 Teilen 3-Amino-phenol versetzt. Die Kupplungsreaktion wird während etwa 12 Stunden unter Einhaltung eines pH-Wertes von 5 bis 5,5 und einer Temperatur von etwa 20°C zu Ende geführt.

Man isoliert die in der Syntheselösung gebildeten erfindungsgemäßen Trisazoverbindungen, die den Formeln (14A) bis (14C) entsprechen, in üblicher Weise durch Aussalzen mit Natriumchlorid oder durch Sprühtrocknung als elektrolytsalzhaltiges (natriumchloridhaltiges) schwarzes Pulver. 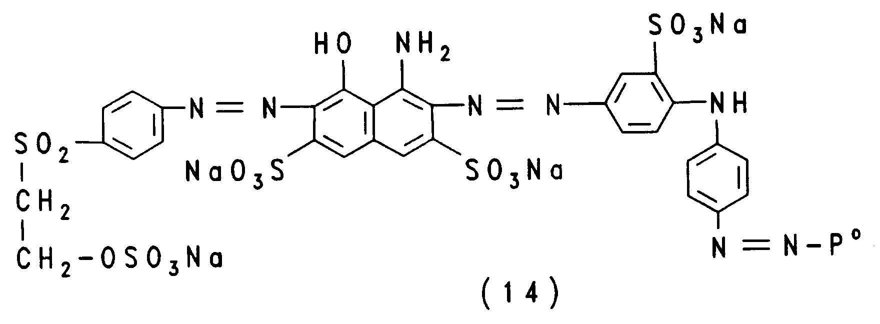
in welcher P^{o} folgende Bedeutung besitzt:
für die Verbindung der Formel (14A): 2-Hydroxy-4-amino-phenyl ,
für die Verbindung der Formel (14B): 4-Hydroxy-2-amino-phenyl ,
für die Verbindung der Formel (14C): 2-Hydroxy-6-amino-phenyl ,
in einem Mischungsverhältnis von etwa 54,4 : 40,2 : 5,4 %.

Die erfindungsgemäße Mischung der erfindungsgemäßen Trisazoverbindungen liefert nach den in der Technik für faserreaktive Farbstoffe übliche Anwendungsbedingungen auf den in der Beschreibung genannten Fasermaterialien, wie insbesondere Baumwolle, Färbungen und Drucke in einem neutral-grauen Farbton.

### Beispiel 4

Zur Herstellung einer erfindungsgemäßen Mischung von erfindungsgemäßen Trisazoverbindungen der allgemeinen Formel (1) verfährt man gemäß einer in den Beispielen 1 bis 3 angegebenen Verfahrensweise, setzt jedoch außer der 1-Amino-8-hydroxy-naphthalin-3,6-disulfonsäure folgende Ausgangsverbindungen in äquimolarer Menge ein: 3-(β-Sulfatoethylsulfonyl)-anilin, 4,4'-Diamino-diphenylamin-2-sulfonsäure und 3-Amino-phenol. Die erfindungsgemäße Mischung enthält die Komponenten entsprechend den Formeln (15A), (15B) und (15C) 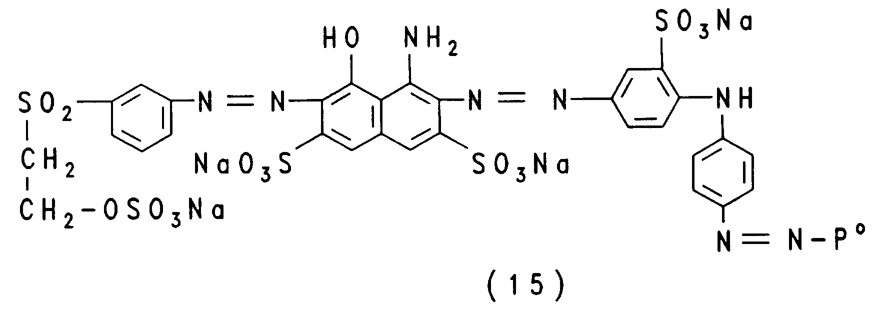
in welcher P^{o} folgende Bedeutung besitzt:
für die Verbindung der Formel (15A): 2-Hydroxy-4-amino-phenyl ,
für die Verbindung der Formel (15B): 4-Hydroxy-2-amino-phenyl ,
für die Verbindung der Formel (15C): 2-Hydroxy-6-amino-phenyl , in einem Mengenverhältnis von etwa 54,4 : 39,1 : 6,5 %. In wäßriger Lösung zeigt die erfindungsgemäße Mischung im sichtbaren Bereich Absorptionsmaxima bei 485 nm und 631 nm.

Man erhält mit der erfindungsgemäßen Mischung dieser erfindungsgemäßen Trisazoverbindungen nach den in der Technik für faserreaktive Farbstoffe üblichen Anwendungsverfahren beispielsweise auf Baumwolle tiefschwarze Färbungen und Drucke mit guten Lichtechtheitseigenschaften.

### Beispiel 5

Zur Herstellung einer erfindungsgemäßen Mischung von erfindungsgemäßen Trisazoverbindungen der allgemeinen Formel (1) verfährt man gemäß einer in den Beispielen 1 bis 3 angegebenen Verfahrensweise, setzt jedoch außer der 1-Amino-8-hydroxy-naphthalin-3,6-disulfonsäure folgende Ausgangsverbindungen in äquimolarer Menge ein: 2,5-Dimethoxy-4-(β-sulfatoethylsulfonyl)-anilin, 4,4'-Diamino-diphenylamin-2-sulfonsäure und 3-Amino-phenol. Die erfindungsgemäße Mischung enthält die Komponenten entsprechend den Formeln (16A), (16B) und (16C) 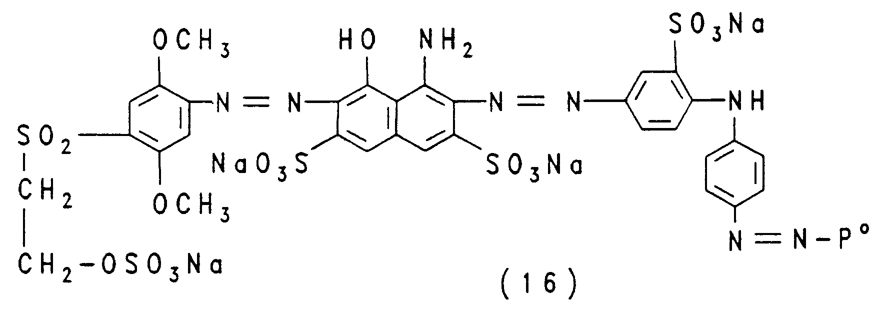
in welcher P^{o} folgende Bedeutung besitzt:
für die Verbindung der Formel (16A): 2-Hydroxy-4-amino-phenyl ,
für die Verbindung der Formel (16B): 4-Hydroxy-2-amino-phenyl ,
für die Verbindung der Formel (16C): 2-Hydroxy-4-amino-phenyl ,
in einem Mengenverhältnis von etwa 53,3 : 40 : 6,7 %. In wäßriger Lösung zeigt die erfindungsgemäße Mischung im sichtbaren Bereich Absorptionsmaxima bei 490 nm und 635 nm.

Man erhält mit der erfindungsgemäßen Mischung dieser erfindungsgemäßen Trisazoverbindungen nach den in der Technik für faserreaktive Farbstoffe üblichen Anwendungsverfahren beispielsweise auf Baumwolle tiefschwarze Färbungen und Drucke mit guten Lichtechtheitseigenschaften.

### Beispiel 6

Zur Herstellung einer erfindungsgemäßen Mischung von erfindungsgemäßen Trisazoverbindungen der allgemeinen Formel (1) verfährt man gemäß einer in den Beispielen 1 bis 3 angegebenen Verfahrensweise, setzt jedoch außer der 1-Amino-8-hydroxy-naphthalin-3,6-disulfonsäure folgende Ausgangsverbindungen in äquimolarer Menge ein: 2-Methoxy-5-methyl-4-(β-sulfatoethylsulfonyl)-anilin, 4,4'-Diamino-diphenylamin-2-sulfonsäure und 3-Amino-phenol. Die erfindungsgemäße Mischung enthält die Komponenten entsprechend den Formeln (17A), (17B) und (17C) 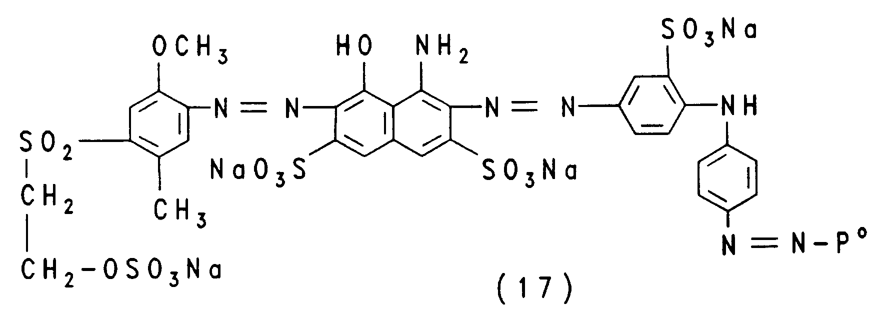
in welcher P^{o} folgende Bedeutung besitzt:
für die Verbindung der Formel (17A): 2-Hydroxy-4-amino-phenyl ,
für die Verbindung der Formel (17B): 4-Hydroxy-2-amino-phenyl ,
für die Verbindung der Formel (17C): 2-Hydroxy-4-amino-phenyl ,
in einem Mengenverhältnis von etwa 53,3 : 30 : 6,7 %. In wäßriger Lösung zeigt die erfindungsgemäße Mischung im sichtbaren Bereich Absorptionsmaxima bei 490 nm und 633 nm.

Man erhält mit der erfindungsgemäßen Mischung dieser erfindungsgemäßen Trisazoverbindungen nach den in der Technik für faserreaktive Farbstoffe üblichen Anwendungsverfahren beispielsweise auf Baumwolle tiefschwarze Färbungen und Drucke mit guten Lichtechtheitseigenschaften.

### Beispiel 7

Zur Herstellung einer erfindungsgemäßen Mischung von erfindungsgemäßen Trisazoverbindungen der allgemeinen Formel (1) verfährt man gemäß einer in den Beispielen 1 bis 3 angegebenen Verfahrensweise, setzt jedoch außer der 1-Amino-8-hydroxy-naphthalin-3,6-disulfonsäure folgende Ausgangsverbindungen in äquimolarer Menge ein: 4-(β-Sulfatoethylsulfonyl)-anilin, 4,4'-Diamino-diphenyl-2-sulfonsäure und 1,3-Dihydroxy-benzol. Die erfindungsgemäße Mischung enthält die Komponenten entsprechend den Formeln (18A) und (18B) 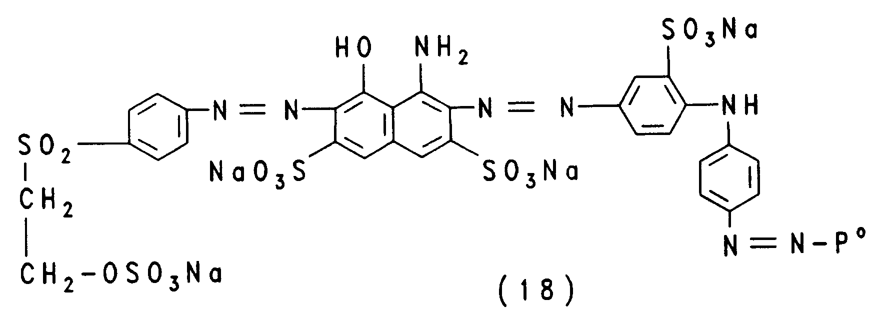
in welcher P^{o} folgende Bedeutung besitzt:
für die Verbindung der Formel (18A): 2,4-Dihydroxy-phenyl ,
für die Verbindung der Formel (18B): 2,6-Dihydroxy-phenyl ,
in einem Mengenverhältnis von etwa 92,5 : 7,5 %. In wäßriger Lösung zeigt die erfindungsgemäße Mischung im sichtbaren Bereich Absorptionsmaxima bei 480 nm und 628 nm.

Man erhält mit der erfindungsgemäßen Mischung dieser erfindungsgemäßen Trisazoverbindungen nach den in der Technik für faserreaktive Farbstoffe üblichen Anwendungsverfahren beispielsweise auf Baumwolle grünschwarze Färbungen und Drucke mit guten Lichtechtheitseigenschaften.

### Beispiel 8

Zur Herstellung einer erfindungsgemäßen Mischung von erfindungsgemäßen Trisazoverbindungen der allgemeinen Formel (1) verfährt man gemäß einer in den Beispielen 1 bis 3 angegebenen Verfahrensweise, setzt jedoch außer der 1-Amino-8-hydroxy-naphthalin-3,6-disulfonsäure folgende Ausgangsverbindungen in äquimolarer Menge ein: 4-(β-Sulfatoethylsulfonyl)-anilin, 4,4'-Diamino-diphenylamin-2-sulfonsäure und 1,3-Diamino-benzol. Die erfindungsgemäße Mischung enthält die Komponenten entsprechend den Formeln (19A) und (19B) 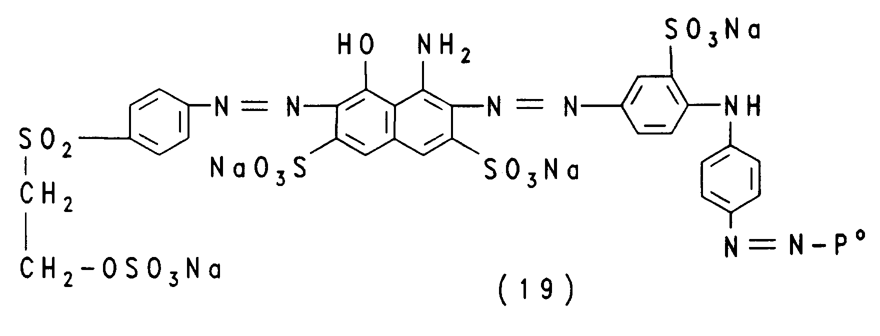
in welcher P^{o} folgende Bedeutung besitzt:
für die Verbindung der Formel (19A): 2,4-Diamino-phenyl ,
für die Verbindung der Formel (19B): 2,6-Diamino-phenyl ,
in einem Mengenverhältnis von 93 : 7 %. In wäßriger Lösung zeigt die erfindungsgemäße Mischung im sichtbaren Bereich Absorptionsmaxima bei 485 nm und 632 nm.

Man erhält mit der erfindungsgemäßen Mischung dieser erfindungsgemäßen Trisazoverbindungen nach den in der Technik für faserreaktive Farbstoffe üblichen Anwendungsverfahren beispielsweise auf Baumwolle schwarze Färbungen und Drucke mit guten Lichtechtheitseigenschaften.

### Beispiel 9

Zur Herstellung einer erfindungsgemäßen Trisazoverbindung der allgemeinen Formel (1) verfährt man gemäß einer in den Beispielen 1 bis 3 angegebenen Verfahrensweise, setzt jedoch außer der 1-Amino-8-hydroxy-naphthalin-3,6-disulfonsäure folgende Ausgangsverbindungen in äquimolarer Menge ein: 4-(β-Sulfatoethylsulfonyl)-anilin, 4,4'-Diamino-diphenylamin-2-sulfonsäure und 4-Sulfo-1,3-diamino-benzol. Die erhaltene erfindungsgemäße Trisazoverbindung besitzt die Formel (20) 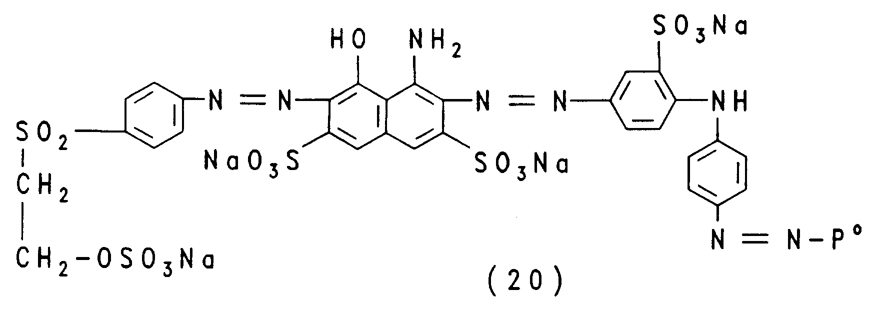
in welcher P^{o} 5-Sulfo-2,4-diamino-phenyl bedeutet. In wäßriger Lösung zeigt die erfindungsgemäße Verbindung im sichtbaren Bereich Absorptionsmaxima bei 484 nm und 630 nm.

Die erfindungsgemäße Trisazoverbindung besitzt sehr gute faserreaktive Farbstoffeigenschaften und liefert nach den hierfür bekannten Anwendungsverfahren beispielsweise auf Baumwolle farbstarke grünschwarze Färbungen und Drucke von guter Lichtechtheit.

### Beispiel 10

Zur Herstellung einer erfindungsgemäßen Trisazoverbindung der allgemeinen Formel (1) verfährt man gemäß Beispiel 3, wobei man folgende Ausgangsverbindungen einsetzt:
4-(β-Sulfatoethylsulfonyl)-anilin, 1-Amino-8-hydroxy-naphthalin-3,6-disulfonsäure, 4,4'-Diamino-diphenylamin-2-sulfonsäure und 4-Sulfo-3-amino-phenol. Die erhaltene erfindungsgemäße Verbindung entspricht der allgemeinen Formel (20), in welcher P^{o} für die 5-Sulfo-4-amino-2-hydroxy-phenyl-Gruppe steht. In wäßriger Lösung zeigt sie im sichtbaren Bereich Absorptionsmaxima bei 484 nm und 630 nm.

Die erfindungsgemäße Trisazoverbindung besitzt sehr gute faserreaktive Farbstoffeigenschaften und liefert nach den hierfür bekannten Anwendungsverfahren beispielsweise auf Baumwolle farbstarke grünschwarze Färbungen und Drucke von guter Lichtechtheit.

### Beispiel 11

Zur Herstellung einer erfindungsgemäßen Trisazoverbindung der allgemeinen Formel (1) verfährt man gemäß Beispiel 3, wobei man folgende Ausgangsverbindungen einsetzt:
2-Methoxy-5-(β-sulfatoethylsulfonyl)-anilin, 1-Amino-8-hydroxy-naphthalin-3,6-disulfonsäure, 4,4'-Diamino-diphenylamin-2-sulfonsäure und 4-Sulfo-1,3-diamino-benzol. Die erhaltene erfindungsgemäße Verbindung entspricht der allgemeinen Formel (10), in welcher P^{o} für die 5-Sulfo-2,4-diamino-phenyl-Gruppe steht. In wäßriger Lösung zeigt sie im sichtbaren Bereich Absorptionsmaxima bei 484 nm und 630 nm.

Die erfindungsgemäße Trisazoverbindung besitzt sehr gute faserreaktive Farbstoffeigenschaften und liefert nach den hierfür bekannten Anwendungsverfahren beispielsweise auf Baumwolle schwarze Färbungen und Drucke von guter Lichtechtheit.

## Patentansprüche

1. Trisazoverbindung entsprechend der allgemeinen Formel (1) und Gemische von Trisazoverbindungen der allgemeinen Formel (1) 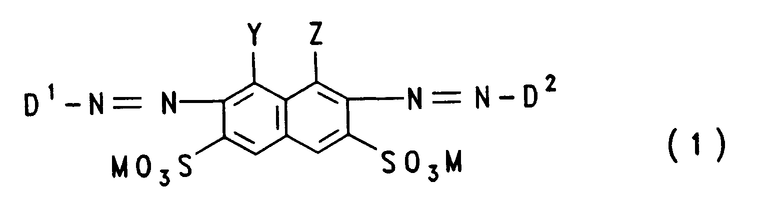 in welcher bedeuten:
M ist Wasserstoff oder ein Alkalimetall;
Y ist Hydroxy oder Amino und
Z ist Hydroxy oder Amino,
wobei im Falle von Y gleich Hydroxy Z gleich Amino und im Falle von Y gleich Amino Z gleich Hydroxy ist;
D¹ ist ein Rest der allgemeinen Formel (2a) 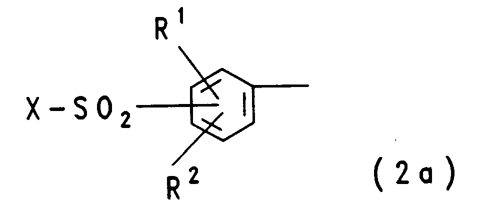 in welcher
R¹ Wasserstoff oder Alkoxy von 1 bis 4 C-Atomen ist,
R² Wasserstoff, Alkyl von 1 bis 4 C-Atomen oder Alkoxy von 1 bis 4 C-Atomen ist und
X Vinyl oder β-Sulfatoethyl ist;
D² ist ein Rest der allgemeinen Formel (2b) 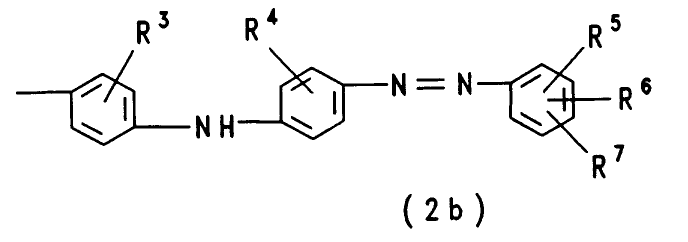 in welcher
R³ Wasserstoff oder Sulfo ist,
R⁴ Wasserstoff oder Sulfo ist,
R⁵ Wasserstoff, Hydroxy oder Amino ist,
R⁶ Wasserstoff, Hydroxy oder Amino ist und
R⁷ Wasserstoff oder Sulfo ist.

2. Gemisch gemäß Anspruch 1, enthaltend Verbindungen entsprechend den nachstehend angegebenen und definierten allgemeinen Formeln (1A), (1B) und (1C) im Mischungsverhältnis von (52-62) : (34-44) : (3-7) Gew.-%, bezogen auf 100 Gew.-Teile der Mischung dieser Verbindungen: 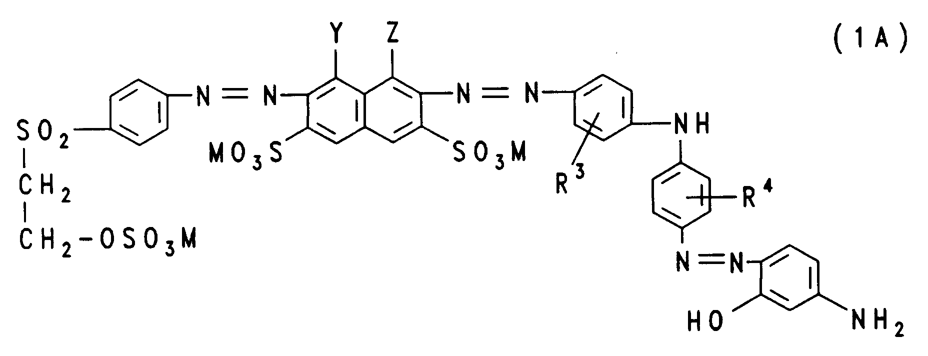 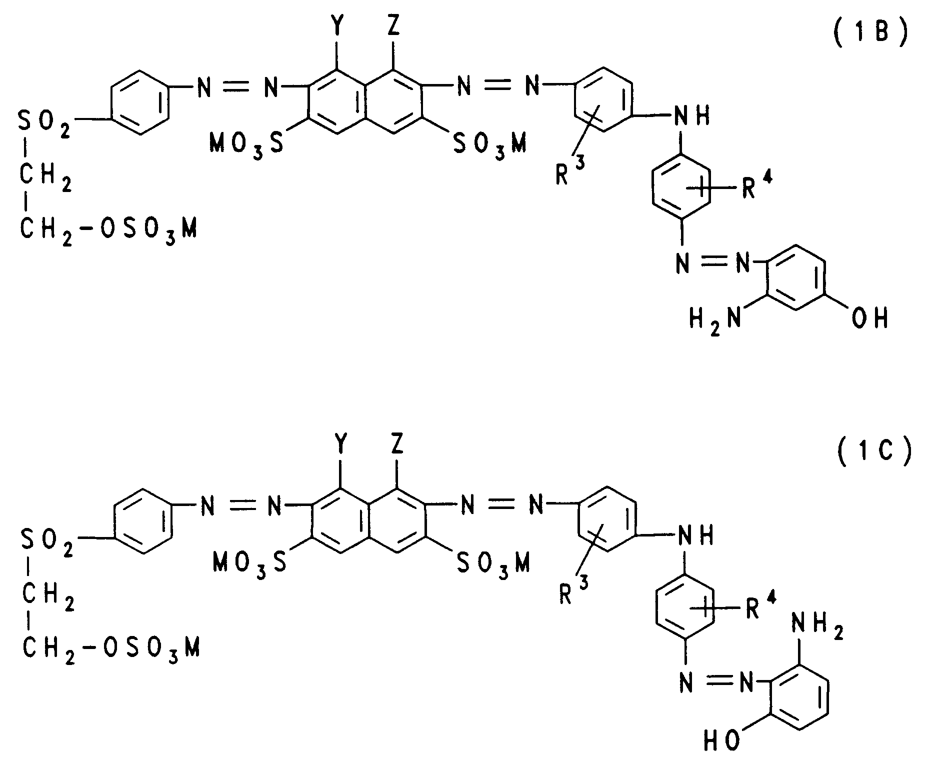 in welchen M, Y, Z, R³ und R⁴ die in Anspruch 1 genannten Bedeutungen haben, wobei jedoch im Falle von R³ gleich Wasserstoff R⁴ gleich Sulfo ist und im Falle von R³ gleich Sulfo R⁴ gleich Wasserstoff ist.

3. Gemisch nach Anspruch 2 von drei Trisazoverbindungen entsprechend der allgemeinen Formel (14) 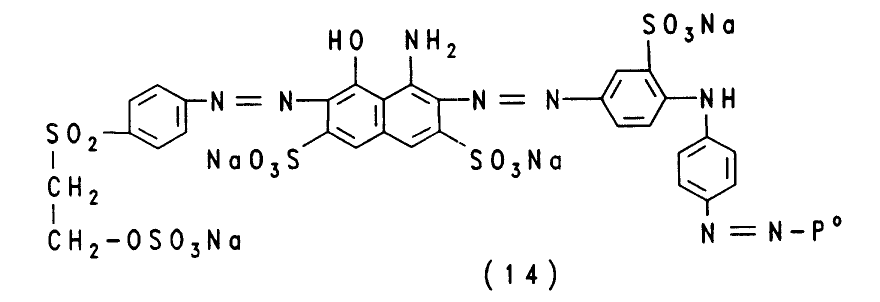 in welcher P^{o} 2-Hydroxy-4-amino-phenyl (Verbindung (14A)), 4-Hydroxy-2-amino-phenyl (Verbindung (14B)) und 2-Hydroxy-4-amino-phenyl (Verbindung (14C)) bedeutet und das Mischungsverhältnis von (14A) : (14B) : (14C) (52-62) : (34-44) : (3-7) Gew.-% beträgt.

4. Gemisch nach Anspruch 3, dadurch gekennzeichnet, daß das Mischungsverhältnis etwa 54,4 : 40,2 : 5,4 Gew.-% beträgt.

5. Verfahren zur Herstellung einer Trisazoverbindung der allgemeinen Formel (1) von Anspruch 1, dadurch gekennzeichnet, daß man ein Diazoniumsalz eines Amins der allgemeinen Formel D¹-NH₂ mit D¹ der in Anspruch 1 genannten Bedeutung und ein Diazoniumsalz eines Amins der allgemeinen Formel D²-NH₂ mit D² der in Anspruch 1 genannten Bedeutung mit der 1-Amino-8-hydroxy-naphthalin-3,6-disulfonsäure kuppelt, oder daß man ein Diazoniumsalz des Amins der allgemeinen Formel D¹-NH₂ mit D¹ der in Anspruch 1 genannten Bedeutung und ein Diazoniumsalz einer Aminoverbindung der allgemeinen Formel (3) 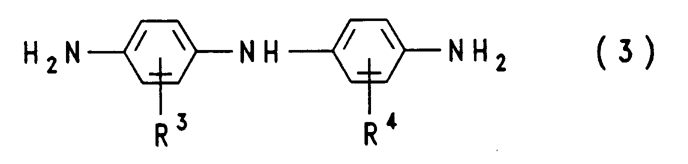 mit R³ und R⁴ der in Anspruch 1 genannten Bedeutung mit der 1-Amino-8-hydroxy-naphthalin-3,6-disulfonsäure kuppelt und die erhaltene Disazoverbindung der allgemeinen Formel (4) 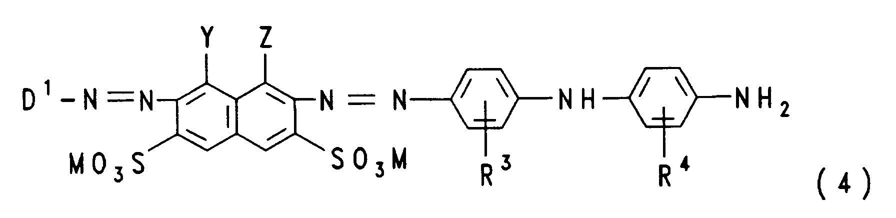 mit D¹, Y, Z, R³ und R⁴ der in Anspruch 1 genannten Bedeutung diazotiert und mit einer Verbindung der allgemeinen Formel (5) 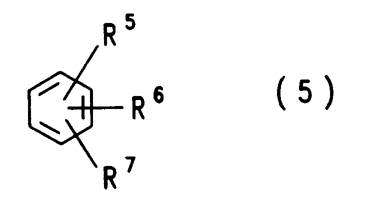 mit R⁵, R⁶ und R⁷ die in Anspruch 1 genannte Bedeutung kuppelt.

6. Verfahren nach Anspruch 5 zur Herstellung eines Gemisches von Trisazoverbindungen der allgemeinen Formel (1), dadurch gekennzeichnet, daß man die Umsetzung mit einem "Eintopf-Verfahren" durchführt.

7. Verwendung einer Trisazoverbindung der allgemeinen Formel (1) oder ein Gemisch derselben zum Färben von Fasermaterialien auf Cellulosebasis natürlichen und synthetischen Ursprungs und als Farbstoff für Tinten.

8. Verfahren zum Färben von Fasermaterialien auf Cellulosebasis natürlichen und synthetischen Ursprungs, dadurch gekennzeichnet, daß man eine Trisazoverbindung der allgemeinen Formel (1) oder ein Gemisch derselben in wäßrigem Medium mit dem Material in Kontakt bringt und die Fixierung der Trisazoverbindung(en) auf dem Fasermaterial mittels Wärme oder mit Hilfe eines alkalisch wirkenden Mittels oder mit Hilfe beider Maßnahmen fixiert.
